Europäisches Patentamt

European Patent Office

Office européen des brevets

(11) Publication number: **0 272 683 B1**

# EUROPEAN PATENT SPECIFICATION

(45) Date of publication of patent specification: **28.07.93**  (51) Int. Cl.⁵: **A61F 13/46**

(21) Application number: **87119040.1**

(22) Date of filing: **22.12.87**

(54) Absorbent pad.

(30) Priority: **22.12.86 US 945934**

(43) Date of publication of application:
**29.06.88 Bulletin 88/26**

(45) Publication of the grant of the patent:
**28.07.93 Bulletin 93/30**

(84) Designated Contracting States:
**AT BE CH DE ES FR GB IT LI LU NL SE**

(56) References cited:
**EP-A- 0 052 403
FR-A- 2 102 456
US-A- 3 375 827
US-A- 3 929 135
US-A- 4 282 874**

(73) Proprietor: **KIMBERLY-CLARK CORPORATION
401 North Lake Street
Neenah, Wisconsin 54956-0349(US)**

(72) Inventor: **Becker, Mollie M.
Lista de Correos
MX-23880 Loreto B.C.S.(MX)**
Inventor: **Sukiennik, Corrine A.
1115 Daniel Court No. 44
Neenah Wisconsin 54956(US)**

(74) Representative: **Diehl, Hermann Dr. et al
Diehl & Glaeser, Hiltl & Partner Flüggen-
strasse 13
W-8000 München 19 (DE)**

## Description

This invention relates to an absorbent pad and refers generally to improvements in absorbent articles, particularly those used for absorption of human exudate. It is particularly suitable for articles used as sanitary napkins.

In the formation of absorbent articles for bodily excretions, one continuing problem is that the bodily excretions are usually directed at one portion of the absorbent pad, whereas the absorptive capacity is spread over a greater area. This may create an early failure problem if the exudate to be absorbed cannot be spread throughout the absorbent. If the fluid does not spread throughout the absorbent, it may run off the edge of the saturated zone, or when the saturated absorbent in the primary flow area is compressed, it may be forced out of the pad and rewet or overflow causing stains or be uncomfortable to wearer.

Another problem to be overcome, in catamenial devices particularly, is that there are fairly large pieces of tissue or blood clots that do not readily pass through a fine cover material. With sanitary napkins it would be desirable if the stained absorbent material could not be viewed after absorption. It also would be desirable if feminine pads and other pads could be visually examined to determine if they were almost saturated. Quite often with the absorption being into a target zone on the top of the pad and then spreading beneath the absorbent, it is not clear from top viewing when the pad is close to being saturated or when the target area is so close to being fully saturated that leakage may soon result.

In US-A-4,423,101 - Willstead and FR-A-1,477,127 - Cartiera Di Cairate, it is disclosed that the cover material may be perforated to aid in the passing of materials through the cover. In US-A-4,480,000 - Watanabe et al. and US-A-3,375,827 - Bletzinger et al., it has been proposed to interpose a layer between the permeable wrapper member and the main portion of the absorbent. Bletzinger et al. utilizes a compressed cellulosic material and indicates that the element spreads the exudate as it is absorbed.

There remains a need for a pad that will provide even distribution of fluid within the pad, a cleaner surface to the pad, better concealment of fluid in the pad and less side leakage.

An object of this invention is to provide a pad for absorbing human exudate that overcomes disadvantages of previous pads.

Another object of the invention is to provide a catamenial pad that provides better catamenial fluid distribution.

An additional object of the invention is to provide a feminine pad that has minimum side staining.

A further object is to provide a pad having a cleaner appearing surface after use.

A further object of the invention is to provide uniform transfer of fluid into the absorbent of a pad for absorption of human exudate.

These and other objects of the invention are generally accomplished by the pad of the independent claim. Further advantageous features are evident from the dependent claims.

The invention provides a pad having a flow zone control layer adjacent the bodyside liner of the pad. The flow zone control layer would be in contact with the absorbent on its lower side and the inner surface of the bodyside liner on its upper surface to provide a fluid passage way from the surface to the absorbent. The flow zone control member would be located only in the middle portion of the pad. The bodyside liner is provided with perforation in the area of the flow zone control layer to aid in transfer of liquids to the flow zone control layer. The pad as is conventional would be provided with an impervious backing member on the side away from the body.

In a preferred embodiment of the invention, the flow zone control layer would be comprised of a meltblown polymer located in the central portion of the pad and not extending to any edge of the pad. The bodyside liner would be a spunbonded material having perforations of a hole size of between about 0.081 (0.032) and about 0.206 cm (0.081 inch) with an open area in the perforated zone of about 22 to about 50 percent. The meltblown flow zone control layer further is preferably of a different color than the absorbent in order to provide indication when the pad needs changing.

The invention provides numerous advantages over prior pads. The pad of the invention provides improved fluid containment. The pad has less side staining and also less leakage at the front and back than previous pads. The pad provides improved removal of fluid from the permeable cover material providing a drier and cleaner surface with less residue on the cover. The pad also provides an improved perception of comfort, cleanliness and absorbency as the covered flow zone control layer provides a perceived improvement in absorbency as it gives the wearer the feeling that the absorbed material is held in the center of the pad.

The flow zone control member also provides an indicator for change time as when fluid begins to extend beyond the flow control member at the surface of the absorbent, it may be considered as an indicator of time to change the pad. The flow zone control means further provides uniform transfer of fluid into the absorbent so as to not overload the center or target area during the early use of the pad. These and other advantages of the invention will be apparent from the description below.

Further object features and advantages of the invention will become evident from the enclosed drawings and the following description.

Figure 1 is a top view of the pad of the invention.

Figure 2 is a sectional view of the pad of the invention taken on cross-sectional line 2-2 of Figure 1.

Figure 2a is an enlarged view of the area indicated as 2a on Figure 2.

Figure 3 is a cross-sectional view of the pad of the invention on cross-sectional line 3-3 of Figure 1.

Figures 4a, 4b, 4c and 4d illustrate the pad of the invention as it is progressively wet by fluid.

Figure 5 illustrates a pad of the invention with an hourglass-shaped flow control zone layer.

Figure 6 illustrates an embodiment of the invention with an oval shape flow transfer layer.

Figure 7 illustrates a pad of the invention with a bow tie-shaped flow transfer layer.

Figure 8 illustrates an embodiment of the invention when the pad is hourglass shaped and the flow zone control layer is also hourglass shaped.

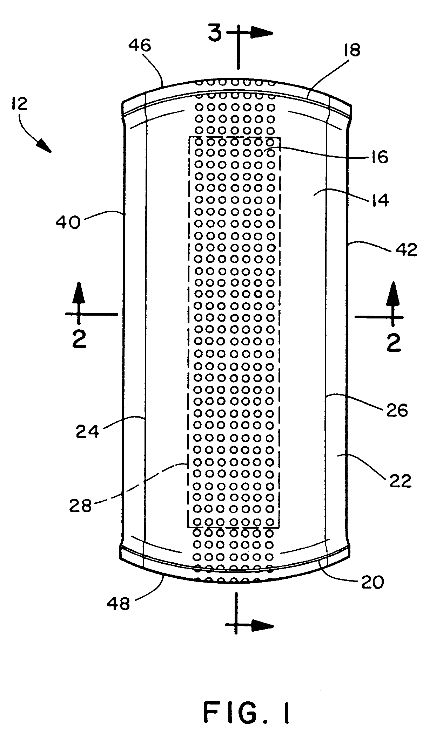

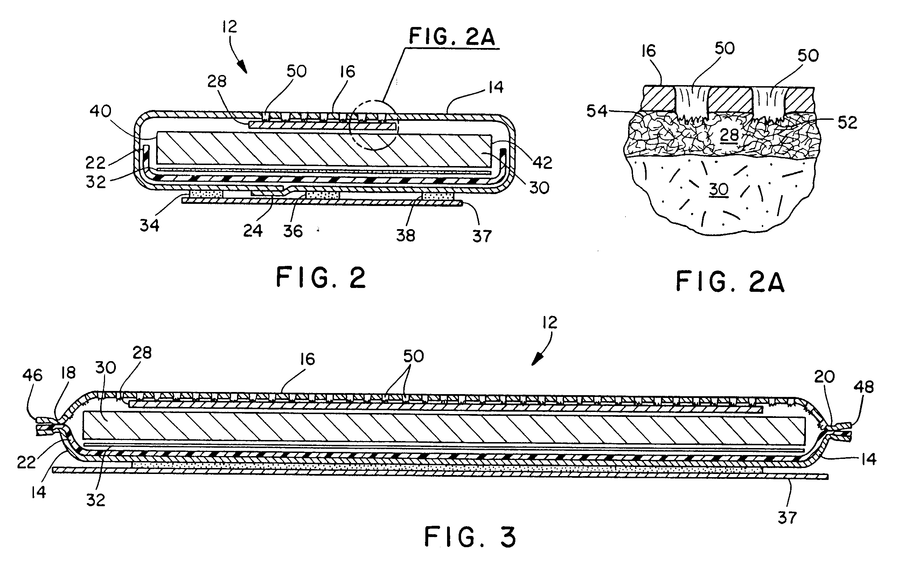

As illustrated in the top view of Figure 1, the pad of the invention 12 is provided with a permeable liner 14 having a perforated longitudinal zone 16. The pad has end seals 18 and 20. The end seals 18 and 20 may be formed by adhesive or ultrasonic sealing. The liner 14 is wrapped under the impermeable backing member 22 and overlapped and sealed at 24. The flow zone control member 28 is below the perforated area 16. As illustrated in Figures 2 and 3, the absorbent 30 is adhesively connected to the backing member 22 by construction adhesive 32. The pad, as illustrated in Figure 2, is provided with a garment attachment adhesive 34 that is protected by a peel strip 37 prior to use. The peel strip 37 is removed prior to attachment of the pad 12 to the wearer's garment (not shown) by the garment adhesive lines 34, 36 and 38. The flow zone control layer 28, preferably formed of a meltblown material, does not extend to the longitudinal edges 40 and 42 or to the ends 46 and 48. The lower sides of the perforations 50 are located in an area generally corresponding to the flow zone control layer 28.

As best shown in the enlarged view of Figure 2a, the perforations 50, as a result of the perforating process, have loose elements 52 that act as sucker feet to become entangled with the fuzzy, hairy or fibrous surface 54 of the flow zone control layer 28. The sucker feet 52 are believed to aid in the transfer of liquid from the bodyside of the pad to the flow zone control layer 28. Further, the fuzzy, hairy, fibrous surface of layer 28 aids in the transfer of the liquid from layer 28 into absorbent 30. Layer 28 is selected such that it will preferentially transfer fluid along its length prior to transferring into layer 30. However, the hairy surface on its lower side aids in transfer to absorbent 30. The hairy surface of the meltblown allows better contact between the cover and the mass of absorbent 30 as the meltblown frictionally by entanglement adheres to the cover and also the absorbent by its hairy surface. Further the perforation sucker feet 52 of the cover aid in the transfer from the cover to the flow zone control means and also to the absorbent if fluid reaches the ends of the pad where the sucker feet would contact the absorbent rather than the flow zone control member.

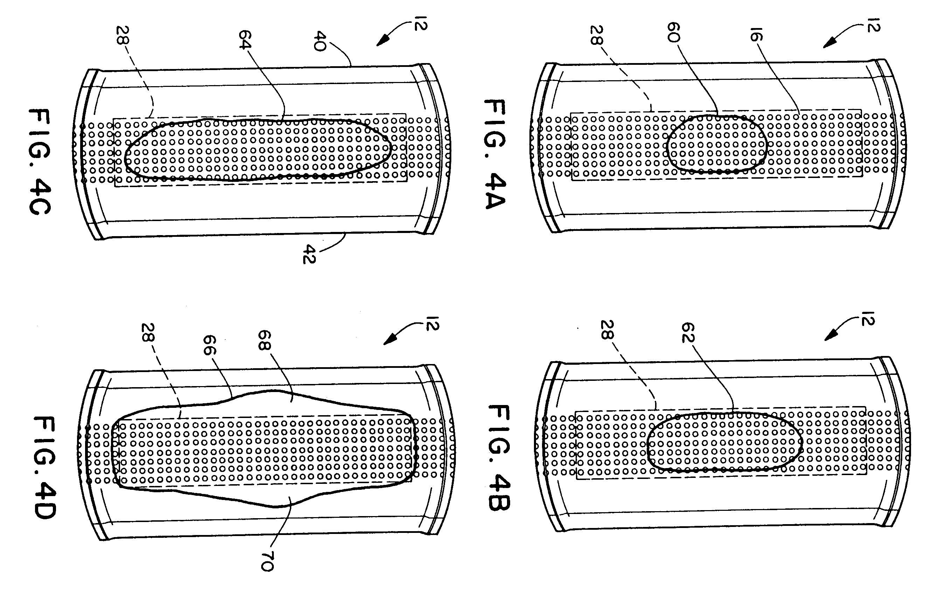

Figures 4a, 4b, 4c and 4d represent the views of the pad of the invention 12 as it is subjected to normal use. Figure 4a illustrates the pad when it is first started to be contacted with bodily exudate such as catamenial fluid or menses. The area 60 represents the initial wetting in the central portion of the pad and the perforated area of 16 overlaying the flow zone control layer 28. Figure 4b shows that as further fluid reaches the pad, the staining extends along the direction of the flow zone control layer 28 and leaves a stained area 62. Figure 4c illustrates the further application of fluid to the pad that has continued to extend along the flow zone control member 28 rather than to the sides of the pad 40 and 42. The stained area 64 now extends almost to the ends of the flow control member 28. Figure 4d illustrates the pad when it is providing visual indication that it should be changed. The stained area 66 has begun to extend outside the flow control layer 28 onto absorbent 30. It is noted that the stain 66 substantially fills the meltblown 28 prior to the color extending into the absorbent in areas 68 and 70. It is noted that generally the fluid in the absorbent 30 will be in a greater area towards the bottom of the pad than will show at the top. Therefore, the pad in Figure 4d is ready for changing but has not leaked to the sides.

Figure 5 illustrates a pad in accordance with the invention. The pad is generally rectangular shaped with the perforated area extending in the longitudinal center of the pad. The pad 80 is provided with a flow zone control member 82 that is hourglass in shape, having a narrowed portion at the crotch 84. This construction may be desirable in that the crotch area is narrowed during wearing of the pad, and it is desirable to transfer fluid to the ends 86 and 88 of the pad.

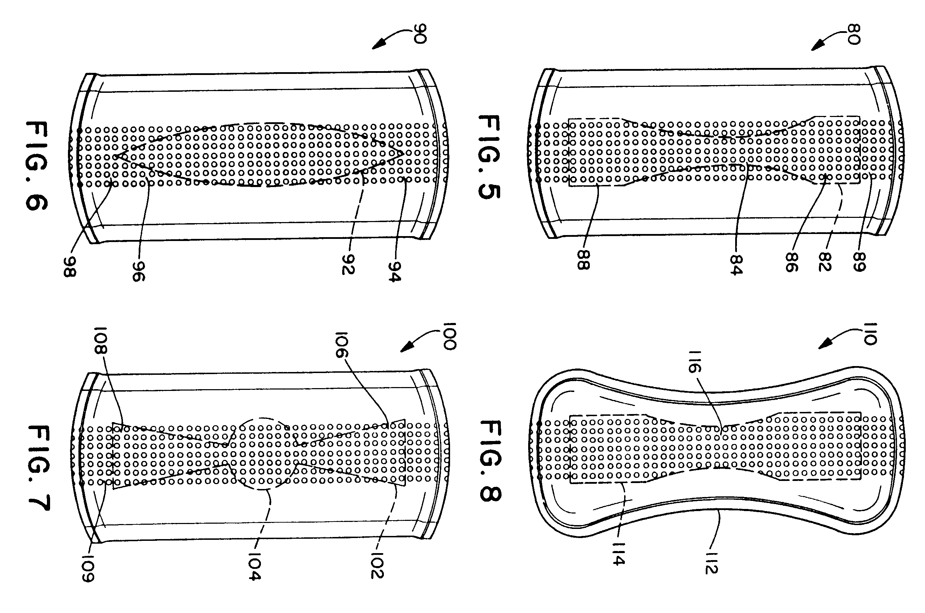

Figure 6 also illustrates a rectangular pad 90 that is provided with an oval flow zone control member 92. The oval flow zone control member is desirable in some instances as it would provide a better appearing pad in that the stained transfer zone would not be visible below as much of the cover, especially near the ends 94 and 96.

Figure 7 illustrates a pad 100 having a flow zone control member 102 in a generally bow tie shape having an enlarged portion 104 in the crotch area and end portions 106 and 108 that are narrower where they contact the center portion 104 and widen towards the ends. This pad is desirable as it is visually pleasing and also indicates to the user a transfer of material to the ends of the pad from target area 104. It is noted that with the pad embodiments of Figures 5, 6 and 7, it is illustrated that the perforated areas 89, 98 and 109 are of uniform width and extend from end to end in the pad. Such areas are the easiest to form as they may be formed continuously in line during production. However, it is possible that, with specialized perforating systems for cover perforating, the perforated area could be formed to correspond to the illustrated flow zone control members and this may be preferred.

Figure 8 illustrates a pad 110 that has a generally hourglass shape with a narrower crotch portion 112. The flow zone control member 114 also is hourglass in shape. The pad further is provided with a narrowed perforated section 116 at the crotch portion. Hourglass-shaped pads are known in the art, and the flow zone control member also would lead to improved performance of such pads.

The cover material forming the liquid-pervious bodyside liner may be any suitable material that is pervious to liquids, nonirritating and which may be perforated. Typical of such materials are bonded carded webs of polyester, polypropylene, nylon or other heat-bondable fibers. Other materials that may be suitable are net materials or finely perforated film webs. A particularly preferred material is spunbonded polypropylene fabric. The most preferred spunbonded polypropylene webs have a weight of between about 18 and about 40 grams per square meter. An optimum weight is between about 30 and about 40 grams per square meter in a white uniform spunbonded material because this material has good masking properties to hide liquid that has passed through it and further retains sufficient strength after perforation in the longitudinal portion that the cover does not tear or fall apart in use. It is also possible that a composite bodyside liner material such as that disclosed in US-A-4,397,644 - Matthews et al. could be utilized as the bodyside member with perforations in the longitudinal center.

The flow zone control layer may be any material that will provide good transfer from the bodyside liner to the flow control layer, provide good fluid transfer along the flow control layer and further, provide good transferring to the lower bulk or mass absorbent of the pad. Typical of such materials are densified fluff, coform, carded webs and creped tissue. Rather than a hairy or fuzzy surface, it is believed that a mechanically roughened, high friction surface webs such as an embossed or creped body wadding or tissue would be suitable for the invention. A preferred material is meltblown polypropylene having a weight of between about 6.8 (0.2) and about 67.8 $g/m^2$ (2.0 ounces per square yard). A most preferred material is a thin layer of meltblown material of between about 33.9 (1.0) and about 67.8 $g/m^2$ (2.0 ounces per square yard) as this material has a frictional hairy, fibrous surface that will adhere to the liner and aid in transfer of liquid from the bodyside liner to the flow zone control layer and also adhere to the absorbent and aid transfer from the flow zone control layer to the bulk absorbent. The preferred meltblown polypropylene is treated to be hydrophylic and will preferentially transfer to a bulk absorbent of devilicated cellulose fibers. The material may be a single sheet of 33.9 (1.0) to 67.8 $g/m^2$ (2.0 ounces per square yard) or folded to achieve that weight. The thickness is generally between about 1 and about 5 millimeters. The meltblown material also may be perforated if desired. The meltblown material also may be colored a different color than the cover material and absorbent. A light blue, pink or peach color has been found to be desirable as these are pleasing feminine colors. The use of a different color than the absorbent also presents a target for the wearer and will indicate when the pad was not placed properly.

The meltblown material has a high surface fiber, fuzzy or hairy surface that, when placed in contact with the perforated cover sheet, has a high coefficient of friction. This high coefficient of friction is believed to aid in maintaining contact of the cover sheet and the flow zone control member. The cover sheet, after perforations, has descended fibers surrounding the raised perforation holes, above referred to as sucker feet, that are believed to become entangled in the surface of the meltblown, resulting in both good contact of the cover and flow zone control member during use and good fluid transfer from the cover to the fluid control zone member. The sucker feet in contact with the meltblown creates a dry-adhesive condition adhering the materials in contact. This coefficient of friction has been found to be preferably greater than about .7. Generally the range is between about .7 and about .12 when utilizing an apertured cover or spunbond material and the meltblown flow zone control member. Generally, the difference between the coefficient of friction of the apertured spunbond and the meltblown as compared with the unapertured spunbond and the same meltblown is between about 15 and about 45 percent lower for the unapertured material than the coefficient of friction measured for the apertured material. The tests of a group of three different unapertured spunbonded materials versus a meltblown material indicated ranges of coefficient of friction from .57 to .73. Coefficient of friction for apertured spunbond with the meltblown material as compared with figures of between about .7 and about 1.1 for the apertured material.

The following table sets forth test results for Coefficient of Friction Tests that compare the frictional properties of meltblown polymer in combination with perforated and unperforated spunbonded cover materials.

| Sample | COF* Forming Side of Meltblown | COF* Wire Side of Meltblown |
|---|---|---|
| A - unapertured | .72 | .67 |
| A - apertured | .90 | .82 |
| B - unapertured | .73 | .66 |
| B - apertured | 1.12 | .96 |
| C - unapertured | .65 | .57 |
| C - apertured | .87 | .76 |

| Sample | COF* Forming Side of Meltblown | COF* Wire Side of Meltblown |
|---|---|---|
| D - unapertured | .63 | .57 |
| D - apertured | .83 | .71 |

Meltblown about 82.15 g/m (2.65 oz./yard) and about 1.2 mm thickness

A = (.8 oz./yd.)- Kimberly-Clark linear drawn Spunbond
    24.8 g/m    Polypropylene
    21.7 g/m
B = (.7 oz./yd.)- Lurgi Spunbonder Polypropylene

C = (.8 oz./yd.)- K-C linear drawn fiber spunbond (modified
    24.8 g/m    cross section) (Lexington Mill)

D = (.8 oz./yd.)- K-C linear drawn fiber spunbond (modified
    24.8 g/m    cross section) (Corinth Mill)

* Average of four tests

The coefficient of friction was determined by use of a Instron Tensile Tester. Utilizing this machine, the meltblown material was fastened to the platform of the tester. A sled of 200 grams had a strip of spunbonded cover material about 11.4 by 7.06 cm (4.5 by 2.78 inches) fastened to the sled. The sled was then pulled across the meltblown material. Tests were done of apertured and unapertured spunbond of the same structure. Testing was performed both on the meltblown wire side of the meltblown and the top or forming side of the meltblown. The coefficient of frictions are slightly higher on the forming side of the meltblown. A reading of the average force required to maintain the sled in motion over the apertured material is read as the force reading. This is divided by the weight of the sled to obtain a coefficient of friction which does not have units.

The absorbent forming the bulk absorbent of the pad may be any desired material. Typical of such materials are rayon, polyesters, coforms and combination of these absorbent fibers, and superabsorbents in combination with fibrous materials. A preferred material is devilicated wood fiber fluff as it is low in cost. The fluff may be wrapped in a tissue wrapping material as is standard in absorbent pad construction. The pad further may contain the cross-linked highly-absorbent polymers ordinarily referred to as superabsorbents either as a separate layer or mixed with the fibers.

The baffle material may be any desirable liquid-impermeable member. It may be vapor-permeable if desired. Typical of such materials are polymer films such as polyethylene film. A preferred material is a polypropylene film as it is low cost and quiet.

The construction adhesives and garment attachment adhesives may be any of those typically utilized in formation of feminine pads, incontinence garments or diapers. Preferred garment attachment would be a single wide band adhesive pattern for maximum attachment strength.

The perforations in the cover are formed in the longitudinal center area generally corresponding with the flow zone control layer. The perforated area generally, for convenience of manufacture, will extend the entire longitudinal length of the pad. However, it would be satisfactory to have perforations only in the zone overlaying the flow zone control layer. Generally the aperturing is such that it provides between about 20 and about 50 percent open area in the apertured area. A preferred amount of aperturing is between about 40 and about 45 percent open area to leave a large amount of remaining integrity in the spunbonded material that is apertured and still provide good transfer of menstrual fluids. The hole size of the perforation generally is between about 0.081 (0.032) and about 0.231 cm (0.091 inch). A preferred hole size is between about 0.152 (0.06) and about 0.203 cm (0.08 inch). The depth of aperture is preferred to be greater than the thickness of the cover liner to create the sucker foot effect that was discussed in reference to the drawings. A variety of regular or irregular aperturing patterns may be utilized.

The width of the central area of longitudinal aperturing may be between about 1.905 (0.75) and about 5.08 cm (2.0 inches) wide, with the wider range being utilized in wider pads and the narrower range being utilized in narrow pads. Generally feminine pads may extend in a width between about 3.81 (1.5) and about 8.89 cm (3.5 inches). The meltblown flow control member generally has a width corresponding to that of the apertured area. In a typical feminine pad, the meltblown flow zone control layer would have a length of between about 11.43 (4.5) and about 16.51 cm (6.5 inches), as this is enough to ensure exposure of the flow zone control laver to the target zone without getting the flow zone control member too close to the edges of the pad.

The cover for bodyside liner may be treated with surfactant if desired to aid in absorption of liquid. However, if the properties of the bodyside web allow its use without treatment with surfactant, a drier feeling cover will result.

While discussed above with prime consideration of catamenial devices, the invention also finds use in other garments and devices for absorption of human exudate. Typical of such other uses are incontinence pads, both those of the smaller shield variety, whose size is only somewhat larger than the typical feminine pad, the larger incontinent garments such as the loincloth-like undergarments and the largest diaper-like adult incontinence garments. The system of the invention also is suitable for use in diapers for infant care.

While described with flat rectangular pads, the system of the invention also is suitable for use with shaped pads, either those shaped with an outer-impermeable baffle of a molded foam, those shaped by elasticized edges or those shaped by having a preformed contoured absorbent structure. The preferred meltblown insert is easily shaped to any desired shape or dimension.

The invention pad, as illustrated, has advantages in the ease of formation. The utilization of a generally rectangular member that is merely laid onto the absorbent prior to covering with the bodyside permeable liner is easy to form. Further, the in-line perforation of a portion of the longitudinal cover is easy to carry out as a line process. There is no need for adhesives or complicated expensive layered cover structures to carry out the instant invention. The invention produces a very effective but low-cost pad.

While the invention has been illustrated with specific examples and materials, there are other variations in materials and form that will be apparent to one of ordinary skill in the art. The scope of the invention is only intended to be limited by the scope of the claims attached hereto.

**Claims**

1. An absorbent pad (12;80;90;100;110) comprising a liquid-impermeable backing member (22), an absorbent member (30) adjacent said backing member (22),
said absorbent pad being characterized by further comprising:
a flow zone control layer (28;82;92;102;114) adjacent the absorbent (30) on the bodyside of said absorbent (30) and a liquid-pervious perforated liner (14) on the bodyside of said pad adjacent said flow zone control layer (28;82;92;102;114).

2. The pad of claim 1 wherein said bodyside liner (14) is perforated in a strip (16;89;98;109;116) in the longitudinal center of said pad (12;80;90;100;110).

3. The pad of one of the preceding claims wherein said bodyside liner (14) is oriented on the pad (12;80;90;100;110) such that liner material (52) displaced during perforating extends toward said flow zone control layer (28;82;92;102;114).

4. The pad of one of the preceding claims wherein said flow zone control layer (28;82;92;102;114) comprises a meltblown polymer located in the central portion of said pad (12;80;90;100;110) and not extending to any edge of said pad.

5. The pad of claim 4 wherein said meltblown is hydrophilic.

6. The pad of one of the preceding claims wherein said perforations have a hole size of between about 0.081 (0.032) and 0.231 cm (0.091 inch).

7. The pad of one of the preceding claims wherein said bodyside liner (14) comprises about 20 to about 50 percent open area.

8. The pad of one of claims 2 to 7 wherein said strip (16;89;98;109;116) is between about 1.90 (0.75) and about 3.81 cm (1.5 inches) wide.

9. The pad of one of the preceding claims wherein said flow zone control layer (28;82;92;102;114) and the perforated areas (16;89;98;109;116) of said pervious liner (14) are between about 1.90 (0.75) and about 3.81 cm (1.5 inches) wide.

10. The pad of one of the preceding claims wherein said flow zone control layer (28;82;92;102;114) is a different color than said absorbent member (30) and said liquid-pervious liner (14).

11. The pad of one of the preceding claims wherein said flow zone control layer (28;82;92;102;114) is between about 11.43 (4.5) and about 16.51 cm (6.5 inches) in length.

12. The pad of one of the preceding claims wherein said flow zone control layer has an hourglass shape (Fig. 5).

13. The pad of one of the preceding claims wherein said pad (110) and said flow zone control layer (114) have an hourglass shape (Fig. 8).

14. The pad of one of claims 1 to 11 wherein said flow zone control layer (102) has a bow tie shape (Fig. 7).

15. The pad of claim 1 wherein said flow zone control layer (92) has an oval shape (Fig. 6).

16. The pad of one of the preceding claims wherein the coefficient of friction between said perforated liner (14) and said flow zone control layer (28;82;92;102;114) is between about .7 and about 1.1.

17. The pad of one of the preceding claims wherein said flow zone control layer (28;82;92;102;114) comprises a meltblown material and said perforated liner (14) comprises a spunbonded material and wherein the coefficient of friction between said meltblown material and said perforated liner (14) is between about 15 and about 45 percent higher than between the spunbonded material prior to being apertured and said meltblown material.

18. The pad of claim 17 wherein the coefficient of friction between said meltblown material and said liner is between about .7 and about 1.1.

**Patentansprüche**

1. Saugfähiges Kissen (12; 80; 90; 100; 110) mit einem flüssigkeitsundurchlässigen Unterlagselement (22), einem an das Unterlagselement (22) angrenzenden saugfähigen Element (30), wobei das saugfähige Kissen desweiteren gekennzeichnet ist durch:
eine an das saugfähige Element (30) angrenzende Fließzonenkontrollschicht (28; 82; 92; 102; 114) auf der körperzugewandten Seite des saugfähigen Elements (30) und eine flüssigkeitsdurchlässige, perforierte Einlage (14) auf der körperzugewandten Seite des Kissens angrenzend an die Fließzonenkontrollschicht (28; 82; 92; 102; 114).

2. Kissen nach Anspruch 1, bei dem die körperseitige Einlage (14) in einem Streifen (16; 89; 98; 109; 116) in der Längsmitte des Kissens (12; 80; 90; 100; 110) perforiert ist.

3. Kissen nach einem der vorhergehenden Ansprüche, bei dem die körperseitige Einlage (14) auf dem Kissen (12; 80; 90; 100; 110) so ausgerichtet ist, daß sich während der Perforierung verschobenes Einlagenmaterial (52) in Richtung der Fließzonenkontrollschicht (28; 82; 92; 102; 114) erstreckt.

4. Kissen nach einem der vorhergehenden Ansprüche, bei dem die Fließzonenkontrollschicht (28; 82; 92; 102; 114) ein schmelzgeblasenes Polymer umfaßt, das in dem Mittelabschnitt des Kissens (12; 80; 90; 100; 110) angeordnet ist und sich nicht bis zu irgendeiner Kante des Kissens erstreckt.

5. Kissen nach Anspruch 4, bei dem das schmelzgeblasene Material hydrophil ist.

6. Kissen nach einem der vorhergehenden Ansprüche, bei dem die Perforierungen eine Lochgröße zwischen etwa 0,081 cm (0,032 in) und 0,231 cm (0,091 in) aufweisen.

7. Kissen nach einem der vorhergehenden Ansprüche, bei dem die körperseitige Einlage (14) etwa 20 bis 50 % offene Fläche aufweist.

8. Kissen nach einem der Ansprüche 2 bis 7, bei dem der Streifen (16; 89; 98; 109; 116) zwischen etwa 1,90 cm (0,75 in) und etwa 3,81 cm (1,5 in) breit ist.

9. Kissen nach einem der vorhergehenden Ansprüche, bei dem die Fließzonenkontrollschicht (28; 82; 92; 102; 114) und die perforierten Bereiche (16; 89; 98; 109; 116) der durchlässigen Einlage (14) zwischen etwa 1,90 cm (0,75 in) und etwa 3,81 cm (1,5 in) breit sind.

10. Kissen nach einem der vorhergehenden Ansprüche, bei dem die Fließzonenkontrollschicht (28; 82; 92; 102; 114) eine andere Farbe als das saugfähige Element (30) und die flüssigkeitsdurchlässige Einlage (14) hat.

11. Kissen nach einem der vorhergehenden Ansprüche, bei dem die Fließzonenkontrollschicht (28; 82; 92; 102; 114) zwischen etwa 11,43 cm (4,5 in) und etwa 16,51 cm (6,5 in) lang ist.

12. Kissen nach einem der vorhergehenden Ansprüche, bei dem die Fließzonenkontrollschicht eine Sanduhrform hat (Fig. 5).

13. Kissen nach einem der vorhergehenden Ansprüche, bei dem das Kissen (110) und die Fließzonenkontrollschicht (114) eine Sanduhrform haben (Fig. 8).

14. Kissen nach einem der Ansprüche 1 bis 11, bei dem die Fließzonenkontrollschicht (102) eine Schleifenform aufweist (Fig. 7).

**15.** Kissen nach Anspruch 1, bei dem die Fließzonenkontrollschicht (92) eine ovale Form aufweist (Fig. 6).

**16.** Kissen nach einem der vorhergehenden Ansprüche, bei dem der Reibungskoeffizient zwischen der perforierten Einlage (14) und der Fließzonenkontrollschicht (28; 82; 92; 102; 114) zwischen etwa 0,7 und etwa 1,1 ist.

**17.** Kissen nach einem der vorhergehenden Ansprüche, bei dem die Fließzonenkontrollschicht (28; 82; 92; 102; 114) ein schmelzgeblasenes Material aufweist, und die perforierte Einlage (14) ein spinngebundenes Material aufweist, und bei dem der Reibungskoeffizient zwischen dem schmelzgeblasenen Material und der perforierten Einlage (14) um etwa 15 bis etwa 45 % höher ist als zwischen dem spinngebundenen Material, bevor es mit Löchern versehen wird, und dem schmelzgeblasenen Material.

**18.** Kissen nach Anspruch 17, bei dem der Reibungskoeffizient zwischen dem schmelzgeblasenen Material und der Einlage zwischen etwa 0,7 und etwa 1,1 liegt.

**Revendications**

**1.** Tampon absorbant (12;80;90;100,110) comprenant un élément support imperméable aux liquides (22), un élément absorbant (30) voisin dudit élément support (22),
   ledit tampon absorbant étant caractérisé en ce qu'il comprend en outre
   une couche de maîtrise de zone d'écoulement (28;82;92;102;114) voisine de l'absorbant (30) sur le côté corporel dudit absorbant (30) et une doublure perforée perméable aux liquides (14) sur le côté corporel dudit tampon au voisinage de ladite couche de maîtrise de zone d'écoulement (28;82;92;102;114).

**2.** Tampon selon la revendication 1, dans lequel ladite doublure côté corporel (14) est perforée selon une bande (16;89;98;109;116) le long du centre longitudinal dudit tampon (12;80;90;100;110).

**3.** Tampon selon l'une des revendications précédentes, dans lequel ladite doublure côté corporel (14) est orientée sur le tampon (12;80;90;100;110) de telle sorte que le matériau de doublure (52) déplacé au cours de l'opération de perforation s'étend en direction de ladite couche de maîtrise de zone d'écoulement (28,;82;92;102;114).

**4.** Tampon selon l'une des revendications précédentes, dans lequel ladite couche de maîtrise de zone d'écoulement (28;82;92;102;114) comprend un polymère obtenu par fusion soufflage disposé dans la partie centrale dudit tampon (12;80;90;100;110) et n'atteignant aucun bord dudit tampon.

**5.** Tampon selon la revendication 4, dans lequel ledit matériau obtenu par fusion soufflage est hydrophile.

**6.** Tampon selon l'une des revendications précédentes, dans lequel lesdites perforations ont une taille de trou comprise entre environ 0,081 (0,032) et 0,231 cm (0,091 pouces).

**7.** Tampon selon l'une des revendications précédentes, dans lequel ladite doublure côté corporel (14) comporte d'environ 20 à environ 50 pour cent de surface ouverte.

**8.** Tampon selon l'une des revendications 2 à 7, dans lequel ladite bande (16;89;98;109;116) a une largeur comprise entre environ 1,90 (0,75) et environ 3,81 cm (1,5 pouce).

**9.** Tampon selon l'une des revendications précédentes dans lequel ladite couche de maîtrise de zone d'écoulement (28;82;92;102;114) et lesdites zones perforées (16;89;98;109;116) de ladite doublure perméable (14) ont une largeur comprise entre environ 1,90 (0,75) et environ 3,81 cm (1,5 pouce).

**10.** Tampon selon l'une des revendications précédentes, dans lequel ladite couche de maîtrise de zone d'écoulement (28;82;92;102;114) est d'une couleur différente de celle dudit élément absorbant (30) et de celle de ladite doublure perméable aux liquides (14).

**11.** Tampon selon l'une des revendications précédentes, dans lequel ladite couche de maîtrise de zone d'écoulement (28;82;92;102;114) a une longueur comprise entre environ 11,43 (4,5) et environ 16,51 cm (6,5 pouces).

12. Tampon selon l'une des revendications précédentes, dans lequel ladite couche de maîtrise de zone d'écoulement a une forme de sablier (Fig. 5).

13. Tampon selon l'une des revendications précédentes, dans lequel ledit tampon (110) et ladite voucher de maîtrise de zone d'écoulement (114) ont une forme de sablier (Fig. 8).

14. Tampon selon l'une des revendications 1 à 11, dans lequel ladite couche de maîtrise de zone d'écoulement (102) a une forme de noeud papillon (Fig. 7).

15. Tampon selon la revendication 1, dans lequel ladite couche de maîtrise de zone d'écoulement (92) a une forme ovale (Fig. 6).

16. Tampon selon l'une des revendications précédentes, dans lequel le coefficient de friction entre ladite doublure perforée (14) et ladite couche de maîtrise de zone d'écoulement (28;82;92;102;114) est compris entre environ 0,7 et environ 1,1.

17. Tampon selon l'une des revendications précédentes dans lequel ladite couche de maîtrise de zone d'écoulement (28;82;92;102;114) comprend un matériau obtenu par fusion soufflage, et ladite doublure perforée (14) est constituée d'un matériau lié au filage et en ce que le coefficient de friction entre ledit matériau obtenu par fusion soufflage et ladite doublure perforée (14) est d'environ 15 à environ 45 % supérieur au coefficient de friction entre le matériau lié au filage avant perforation et ledit matériau obtenu par fusion soufflage.

18. Tampon selon la revendication 17 dans lequel le coefficient de friction entre ledit matériau obtenu par fusion soufflage et ladite doublure est compris entre environ 0,7 et environ 1,1.

FIG. 1

FIG. 2

FIG. 2A

FIG. 3

EP 0 272 683 B1

FIG. 4A

FIG. 4B

FIG. 4C

FIG. 4D

FIG. 5

FIG. 8

FIG. 6

FIG. 7